# EUROPEAN PATENT SPECIFICATION

(11) **EP 1 726 316 B1**
(45) Date of publication and mention of the grant of the patent: **16.12.2009**
(21) Application number: 05011238.2
(22) Date of filing: 24.05.2005
(51) Int. Cl.: A61L 15/26

(54) **Wound care system comprising a polyalkylene carbonate film dressing**
Wundbehandlungsystem mit einem Filmpflaster aus Polyalkylencarbonat
Système de soin comprenant un pansement sous forme de film à base de polyalkylène de carbonate

(43) Date of publication of application: 29.11.2006
(73) Proprietor: MEDPAK, LLC, Allentown, Pennsylvania 18104 (US)
(72) Inventor: Robins, Perry, New York, NY 10016 (US); Sant'angelo, Joseph G., Allentown PA 18104 (US)
(74) Representative: Marx, Lothar

(56) References cited:
- EP-A- 0 473 867
- US-A- 4 142 021
- US-A- 5 725 491
- US-A1- 2005 208 153
- US-B1- 6 909 027
- PATENT ABSTRACTS OF JAPAN vol. 014, no. 299 (C-0733), 27 June 1990 (1990-06-27) & JP 02 097525 A (MITSUI PETROCHEM IND LTD), 10 April 1990 (1990-04-10)

## Description

This invention pertains to a wound care system, including a unique dressing and the composition constituting the dressing.

### BACKGROUND OF THE INVENTION

This invention relates to wound care covering both external wounds (skin, etc) as well as internal wounds (organs, etc). There are many aspects to wound care, both medical and economic. The shorter the treatment time, the more economical the process and the greater the patient comfort. Treatment and nursing costs are up to 10 times higher than supply costs. Some complications that increase treatment time are infections, non-optimal wound care dressing, longer healing and closure times. These are affected by the wound care system used. The most common wound dressings have been gauze and tape. Subsequently, multi-layered systems have been designed with a film backing. Liquid bandages that provide a film covering of the wound have also been suggested. These wound coverings provide some of the desired protection to wounds, but do not provide the optimal environment for enhanced wound healing and minimal scarring.

Gauze dressings may not completely protect the wound from water, bacteria, dirt, external oxygen and other foreign matter. Gauze can stick to wounds causing wound damage during dressing changes, increasing the probability of infection and lengthening healing time.

Multi-layered dressings are an improvement over gauze dressings, however they can be stiff and bulky, can be susceptible to channeling permitting bacteria and foreign material to enter wounds. Generally these dressing have a layer for absorbing wound exudate and need to be changed during wound healing. Damage can occur to the wound due to sticking of the dressing to the wound. These multi-layered dressings can have a film backing. Typically polyurethane is used. It does not provide the desired barrier to external oxygen to protect the wound from the external oxygen during the early stages of healing. Some multi-layered dressings may provide some low level of oxygen barrier due simply to the dressing and its thickness.

Previously suggested liquid bandages are usually polymer solutions dissolved in organic solvents. The solution is applied directly to the wound, the solvent is evaporated and or precipitated, or the solution is coagulated in situ leaving a film covering over the wound. These films can be nitrocellulose polymers or cyanoacrylates. These films do not provide oxygen barrier properties to the wound site. Since they are not elastomers, they do not provide elasticity and recovery to the film. Due to their high softening temperatures they do not permit the film to flow as a result of the skin/body heat. The nitrocellulose films have a tendency to form cracks when placed on a site that is flexing, e.g. base of the thumb or any other anatomical body part that has movement. These cracks then destroy the protective covering of the wound. These films can also give off undesirable chemical odors. None of the above wound coverings provide all of the desired combination of properties needed for enhanced wound healing, such as oxygen barrier, elasticity and recovery, low glass transition temperatures for improved conformity to the site, non-sticking to the wound due to enzyme degradation of the polymer at the wound site giving off carbon dioxide and water.

Polycarbonates have been given as one of many polymers to be used as a film. They do not work as is taught in this application. Polycarbonates are different polymers than polyalkylene carbonates.

In wounds, new blood vessels grow from zones rich in oxygen toward those with less oxygen. (Knighton D et al. "Oxygen tension regulates the expression of angiogenesis factor by macrophages". Science1983 221:1283-5.) Normal tissue has an oxygen tension of about 40 mm of Hg. Angiogenesis are stimulated at about 1-10mm Hg relatively hypoxic conditions. (Hunt TK, et al. "Wound microenvironment in Cohen IK et al. (eds) Wound healing: Biochemical and Clinical Aspects. "Philadelphia, WB Saunders, 1992, pp. 274-281); and (Pai MP et al. "Effect of varying oxygen tensions on healing of open wounds." Surg Gynecol Obstet 1972; 135: 756-758); and (Storch, TG et al. "Oxygen concentration regulates the proliferation responses of human fibroblast to serum and growth factors. Exp Cell Res 1988; 175: 317-325)

Dressings impermeable to outside oxygen cause angiogenesis to proceed most rapidly. Early oxygen depravation and later oxygen supplementation are conducive to healing. Reduced wound pH also contributes to angiogenisis. A low pH is maintained by preventing wound tissues from losing all of their carbon dioxide.

Some of the problems related to certain wound care systems in prior art methods include:
- non-optimal(higher) gaseous oxygen environment around the wound as a result of wound dressings permitting outside oxygen to enter the wound area in the early stages;
- lack of proper (adequate) moisture;
- bacterial invasion into wound area due to poor bandage adhesion and/or coverage, and film coverings which are not elastic and therefor form cracks, channels and wrinkles leading to the wound;
- non-optimal removal of exudate related to insufficient water vapor transmission from the wound;
- improper mechanical pressures on wound;
- contamination of gauze bandages due to handling;
- damage to the cells in the healing process by gauze bandages that stick to wounds and cause damage, particularly during dressing changes by peeling off epidermal cells and interfering with healing;
- stiff dressings which may be flexible but are not elastic so as to conform tightly to body shapes during movement;
- loss of carbon dioxide from wound tissues leading to high pH and alkalosis;
- skin sensitivity to tapes causing rashes;
- chemical odors of varying degrees; and
- failure of other film dressings to incorporate all of the key properties needed in a solitary film.

JP-A-02 097525 A discloses the production of polyalkylene carbonate wherein the polymer is applicable to general moldings, films, binders, medical materials, etc. The specific in situ film dressing according to the present invention is not described.

EP-A-0 473 867 describes bioabsorbable copolymers comprising p-dioxanone and AA-BB-type polyalkylene carbonates for sutures and suture coatings.

The present invention provides an interactive wound care system that addresses the above problems. Our system provides an occlusive covering and interaction with the wound during the healing process, thereby providing the optimum gas and liquid environment for enhancing wound healing, improving patient comfort and reducing costs.

### BRIEF SUMMARY OF THE INVENTION

Enhanced wound healing and minimal scarring occur when the wound covering permits the required concentration around the wound of oxygen, water moisture, exudate, carbon dioxide and bacteria.

A recently developed family of polymers, called polyalkylene carbonates (PAC), are utilized to design the optimum environment in and around the wound. These polymers are produced by reacting carbon dioxide with epoxides. (Inoue S, "Organic and Bio-Organic Chemistry of Carbon Dioxide" Halsted Press, New York , pp167-176, 1982) The resulting properties of the polymer are a function of the epoxide selected.

Polymers can be produced with properties that range from soft elastomeric with low glass transition temperatures (15 °C to 25 °C to 40 °C), to hard stiff polymers with high glass transition temperatures, e.g. 132°C. Intermediate properties can be produced by chemical(terpolymers) and physical (blends)means. These polymers then can be selected to provide various degrees of oxygen barrier to the wound from exterior oxygen, as well as carbon dioxide transmission from the wound for enhanced wound healing.

Films made from these polymers adhere to skin forming a barrier to outside dirt, water, and bacteria and allow water vapor permeability from the wound permitting excess exudate and bacteria also to leave the wound, through evaporation, thereby reducing wound pressures and infections.

These films will not adhere to wounds, thereby permitting frequent dressing changes without disturbing the normal healing process of the wound. Additionally, these polymers can be dissolved in a number of biologically acceptable solvents providing solutions of one or more polyalkylene carbonates with one or more solvents to permit designing the appropriate film, foam, or gel composition. These polymers can also be produced as water based emulsions. These solutions or emulsions can then be brushed or sprayed around the wound, forming a conforming protective and interactive film to enhance wound healing. Other methods of application such as gels from squeeze tubes, melt formed films or liquid solutions rolled on or spread with a squeegee or spatula can be used. Other additives may be dissolved or dispersed in these solutions to permit design of a chemical medical system to optimize wound healing. Medicaments that can be added to the polyalkylene fluid include, for example, any of the well-know anti-bacterial agents (such as erythromicin and clindamycin), steroids (such as clobetasol, triamcinolene, and fluocinonide), anti-fungal agents (such as cicloprox [Penlac], griseofulvin, and terbinafine [Lamisil]), immunomodulators, anti-microbial agents, anti-acne agents, and anti psoriasis agent s. Wounds may first be pretreated with medicaments, etc. and then sprayed over with the protective film covering etc. The object of this invention is to address problems inherent in other wound care dressings by properly designing the appropriate polyalkylene carbonate polymer or combination of polyalkylene carbonate polymers to solve the problems of current dressings.

Physical chemical properties of one or more of this family of polyalkylene carbonate polymers which can be selectively utilized are:
- clear, amorphous, thermoplastic;
- glass transition temperatures in the range of from 15°C to 132°C;
- excellent adhesion to skin , non-sticking to wound;
- soft, elastomeric polymers with good recovery, to hard engineering polymers;
- enzyme degrades or bum cleanly into primarily carbon dioxide and water;
- excellent oxygen barrier;
- semi-permeable to water vapor;
- low cost
- quick drying with no odor;
- soluble in a wide range of solvents from low boiling to high boiling;
- low glass transition temperature of polyethylene carbonate, e.g. 25 °C;
- makes for a soft, flexible and elastic bandage, which is softened by the skin and body temperature promoting conformability to body shapes in motion;
- controllable oxygen permeability;
- carbon dioxide semi-permeability;
- barrier to out side water, dirt, and bacteria;
- can be produced as water based emulsions; and
- excellent self adhesion of films.

### DETAILED DESCRIPTION OF THE INVENTION

The problems set are solved according to the subjet matter of claim 1.

To use this invention, the polyalkylene carbonate polymer or polymers is/are applied to animal tissue (e.g., over a wound) in a fluid form. Thus, for example, the polyalkylene carbonate can be dissolved in a biocompatible solvent or solvents. ride, dichloroethane, propylene carbonate, dimethylformamide, N-Methyl pyrrolidone, acetone, ethyl acetate, tetrahydrofuran, methyl ethyl ketone as well as other ketones, esters, ethers, etc. The polymer concentration is a function of the delivery system.

As a general rule, it is preferable to use polyalkylene carbonates wherein the alkylene component contains from about two to about nine carbon atoms. It is particularly preferred to use polyalkylene carbonates wherein the alkylene component is one of ethylene, propylene and butylene.

Some of the preferred solvents include methylene chloride and acetone, while generally alcohols and other well-known organic solvents are operable. When the polyalkylene carbonate is polyethylene carbonate it is preferred to use methylene chloride as the solvent, while it is preferred to use acetone as the solvent when the polyalkylene carbonate is polypropylene carbonate.

The wound is first cleaned, and then any of the following methods of application are used:
- If a spray can (aerosol) or bottle spray is used, then a lower concentration of polymer is used to provide the proper viscosity for spraying and film forming on and around the wound. This concentration would also be a function of the solvent selected and molecular weight. Polymer concentrations in this application usually range of from 5 to 35% by weight of the solution.
- If a brush, Q-tip, eye-dropper or rod are used, then an intermediate polymer concentration is used with proper viscosity to prevent the solution from running away from the wound. Polymer concentrations in this application usually range of from 5 to 50% by weight of the solution.
- If a gel or squeeze tube is used, the polymer concentration can higher. The gel can e applied directly and spread out to form a film on and around the wound. Polymer concentration in this application is usually in the range of from 20 to 60% by weight of the gel.
- If a melt film is to be used, dispensed from a melt film forming device, the polymer concentration can be relatively high, e.g. up to about 100% by weight polymer minus any additives, e.g., absorbents, moisturizers, medications, plasticizers, etc.
- If an emulsion is used, e.g., a water-based emulsion, the polymer concentration can be maximized based upon other chemicals in the system. Polymer concentration can be in the range of from 10 to 60% by weight based upon the emulsion.

The films produced from any of the above methods have excellent adhesion to itself. Therefore, these films can be made to wrap completely around certain body parts, and to adhere to itself. Spraying on a film can provide better protection around wounds in odd shaped (irregular shapes) of the body, by providing a complete seal around the wound.

The use of polyethylene carbonate with excellent oxygen barrier properties, low Tg of about 25 °C., very high elongation and recovery , flexibility and elasticity provides excellent conformity and protection to irregular body shapes. The low Tg, permits body skin temperatures to soften the polymer further and better conform to irregular shapes, increasing the patients comfort and providing excellence protection to the wound. Film thickness can be from about 0.01mm (0.25 mils) to greater than about 0.08mm (3.0 mils) e.g., about 0.09mm (3.5 mils).

In the early stages of healing in certain types of wounds, external oxygen is not desirable. By blocking external oxygen and permitting the internal oxygen in blood vessels from within to assist in the wound healing, wounds heal more rapidly and with less scarring. Prior patents did not recognize this important role of oxygen exclusion. They taught the opposite, "an adequate exchange" of oxygen and or films that are "semi -permeable" to oxygen. Kay, US 5,713,842, teaches allowing "the free diffusion of oxygen, water vapor, and other gases through their molecular matrices," with polyurethane films being preferred. Tipton et al. US 5,725,491 teaches "it is preferred that the size and number of pores of the film dressing facilitate diffusion of nutrients, oxygen, water and biologically active agents."

Examples in prior patents of polymers that can be used do not include polyalkylene carbonates.

Wounds treated with oxygen permeable films produce lower recovery rates. Occlusive dressings have a lower infection rate than non-occlusive dressings. Therefore, by using polyethylene carbonate film as the polymer of choice we combine:
- oxygen barrier;
- low Tg, from 15 to 40 °C., preferably from 20 to 30 °C., and most preferably about 25 °C., allowing body temperatures to soften the polymer and flow to produce excellent conformity to irregular body surfaces, excellent adhesion to skin around the wound, and improved comfort for the patient;
- foreign material barrier;
- water proofing from external water;
- elastomeric properties producing non-cracking film from surface movement;
- good insulating properties;
- one component system - easy to apply, may be sold over the counter;
- good skin adhesion to keep out bacteria and foreign matter;
- excellent self adhesion-for complete wrap around or multiple layers, if needed;
- clear film for wound observation, if desired;
- enzyme degradability of polymer at wound /film surface interface forming carbon dioxide and water to enhance bandage removal and wound healing;
- oil resistant, acid and base resistant, therefore not affected by body oils or fluids;
- abrasion resistant, not easily damaged by external forces;
- chemical/medical additives - good bonding , dispersion or solution;
- does not stick to wounds, permitting frequent bandage changes without damaging repair cells in the wound, which reduces odors and promotes healing;
- film is permeable to water vapor, helping to remove exudate and bacteria;
- semi permeable to carbon dioxide helping to maintain a low pH and preventing alkalosis;
- with methylene chloride as the solvent, film is quick drying and has no residual odor;
- indefinite shelf life; and
- low cost wound cover.

A preferred method of using this invention is to produce a solution containing from 10 to 15% by weight of the solution of polyethylene carbonate in methylene chloride. The wound is washed or pretreated, then dried. It is then coated with the polymer solution, brush or spray, and allowed to dry. The drying process is a matter of minutes due to the low boiling point of the solvent, i.e. 39.7 °C. The skin temperature is about 33 °C., body temperature about 37 °C., promoting evaporation of the solvent, and flow of the polymer, which has a glass transition temperature of 20-25 °C. The wound healing process is observed through the clear film. If dressing changes are required, the film is easily removed and new film applied. The film is removed when the healing is completed.

In certain other type wounds or in the later stages of certain wounds, external oxygen may be desired and, therefore, polypropylene carbonate can be used, since it is not a good oxygen barrier. By blending polypropylene carbonate and polyethylene carbonated either physically or chemically (terpolymer), intermediate properties can be obtained to optimize wound heating., as a function of wound type.

There are no other polymer families that can incorporate the unique broad range combination of physical /chemical properties obtainable with this new family of polymers, polyalkylene carbonates. They can be "tailored" to fit the application, thereby providing a total healing system that enhances wound healing, reduces scarring, adds to patient comfort and reduces costs.

Wound healing is a complex process. Significant, valuable medical knowledge has been developed which can enhance wound care. Polyalkylene carbonate polymers have a broad range of properties that can effectively be utilized with the current medical knowledge to produce a new generation of wound coverings. They also have the potential to be tailored to meet new medical requirements as new knowledge in wound care is developed.

## Claims

1. Polyalkylene carbonate for use in forming an in situ film wound care dressing on animal tissue, which comprises preparing an application fluid comprising a polyalkylene carbonate, applying the fluid to animal tissue and thereby forming a film on the animal tissue.

2. Polyalkylene carbonate for use in forming an in situ film dressing of Claim 1 wherein the polyalkylene carbonate is placed into fluid form by raising the polyalkylene carbonate to a temperature at least as high as its glass transition temperature and pressing the fluid polyalkylene carbonate to the animal tissue.

3. Polyalkylene carbonate for use in forming an in situ film dressing of Claim 1 wherein the polyalkylene carbonate is placed in fluid form by forming a suspension of the polyalkylene carbonate in a biocompatible medium, applying the fluid polyalkylene carbonate to the animal tissue and evaporating the biocompatible medium.

4. Polyalkylene carbonate for use in forming an in situ film dressing of Claim 1 wherein the polyalkylene carbonate is placed in a fluid form by forming an emulsion of the polyalkylene carbonate in a biocompatible medium, applying the fluid polyalkylene carbonate to the animal tissue and evaporating the biocompatible medium.

5. Polyalkylene carbonate for use in forming an in situ film dressing of Claim 1 wherein the polyalkylene carbonate is placed in fluid form by forming a solution of the polyalkylene carbonate in a biocompatible solvent, applying the fluid polyalkylene carbonate solution to the animal tissue and evaporating the biocompatible solvent.

6. Polyalkylene carbonate for use in forming an in situ film dressing of Claim 1 wherein the alkylene component of the polyalkylene carbonate contains from two to nine carbon atoms.

7. Polyalkylene carbonate for use in forming an in situ film dressing of Claim 1 wherein the alkylene component of the polyalkylene carbonate is selected from the group consisting of ethylene, propylene and butylene.

8. Polyalkylene carbonate for use in forming an in situ film dressing of Claim 5 wherein the content of the polyalkylene carbonate in the solution is from 5% to 50% by weight based on the solution and the solution is applied onto the animal tissue.

9. Polyalkylene carbonate for use in forming an in situ film dressing of Claim 3 wherein the content of the polyalkylene carbonate in the suspension is from 10% to 60% by weight based on the suspension and the suspension is applied onto the animal tissue.

10. Polyalkylene carbonate for use of Claim 1 wherein the fluid polyalkylene carbonate also contains a medicament.

11. Polyalkylene carbonate for use of Claim 10 wherein the medicament is selected from the groups consisting of anti-bacterial agents, steroids, anti-fungal agents, immunomodulators, anti-microbial agents, steroids, anti-acne agents, and anti-psoriasis agents.

12. Polyalkylene carbonate for use in forming a film dressing on animal tissue of Claim 1 which comprises a polyalkylene carbonate having a glass transition temperature (Tg) of from 15 to 40 °C. in a fluid form, which fluid polyalkylene carbonate adheres to animal tissue.

13. Polyalkylene carbonate for use of Claim 12 wherein the polyalkylene carbonate is placed in fluid form by raising the polyalkylene carbonate to a temperature at least as high as its glass transition temperature.

14. Polyalkylene carbonate for use of Claim 5 wherein the alkylene component of the polyalkylene carbonate is ethylene and the biocompatible solvent for polyethylene carbonate comprises methylene chloride.

15. Polyalkylene carbonate for use of Claim 5 wherein the alkylene component of the polyalkylene carbonate is propylene and the biocompatible solvent for polypropylene carbonate comprises acetone.

16. Polyalkylene carbonate for use of Claim 5 wherein the polyalkylene carbonate is polyethylene carbonate having a glass transition temperature of from 20 to 25°C, the biocompatible solvent is methylene chloride and the polyethylene carbonate is present in the solution in a concentration of from 10 to 15% by weight based upon the solution.

## Patentansprüche

1. Polyalkylencarbonat zur Verwendung in der Bildung eines in situ-Filmwundversorgungsverbands auf Tiergewebe, welche das Herstellen eines Aufbringungsfluids, umfassend ein Polyalkylencarbonat, das Aufbringen des Fluids auf Tiergewebe und dabei das Bilden eines Films auf dem Tiergewebe umfasst.

2. Polyalkylencarbonat zur Verwendung in der Bildung eines in situ-Filmverbands nach Anspruch 1, wobei das Polyalkylencarbonat in Fluidform platziert wird, indem das Polyalkylencarbonat auf eine Temperatur von mindestens so hoch wie seine Glasübergangstemperatur erhöht wird und das fluide Polyalkylencarbonat auf das Tiergewebe gepresst wird.

3. Polyalkylencarbonat zur Verwendung in der Bildung eines in situ-Filmverbands nach Anspruch 1, wobei das Polyalkylencarbonat in Fluidform platziert wird, indem eine Suspension des Polyalkylencarbonats in einem biokompatiblen Medium gebildet wird, das fluide Polyalkylencarbonat auf das Tiergewebe aufgebracht wird und das biokompatible Medium abgedampft wird.

4. Polyalkylencarbonat zur Verwendung in der Bildung eines in situ-Filmverbands nach Anspruch 1, wobei das Polyalkylencarbonat in einer Fluidform platziert wird, indem eine Emulsion des Polyalkylencarbonats in einem biokompatiblen Medium gebildet wird, das fluide Polyalkylencarbonat auf das Tiergewebe aufgebracht wird und das biokompatible Medium abgedampft wird.

5. Polyalkylencarbonat zur Verwendung in der Bildung eines in situ-Filmverbands nach Anspruch 1, wobei das Polyalkylencarbonat in Fluidform platziert wird, indem eine Lösung des Polyalkylencarbonats in einem biokompatiblen Lösungsmittel gebildet wird, die fluide Polyalkylencarbonatlösung auf das Tiergewebe aufgebracht wird und das biokompatible Lösungsmittel abgedampft wird.

6. Polyalkylencarbonat zur Verwendung in der Bildung eines in situ-Filmverbands nach Anspruch 1, wobei die Alkylenkomponente des Polyalkylencarbonats zwei bis neun Kohlenstoffatome enthält.

7. Polyalkylencarbonat zur Verwendung in der Bildung eines in situ-Filmverbands nach Anspruch 1, wobei die Alkylenkomponente des Polyalkylencarbonats aus der Gruppe ausgewählt ist, welche aus Ethylen, Propylen und Butylen besteht.

8. Polyalkylencarbonat zur Verwendung in der Bildung eines in situ-Filmverbands nach Anspruch 5, wobei der Gehalt des Polyalkylencarbonats in der Lösung 5 Gew.-% bis 50 Gew.-%, bezogen auf die Lösung, beträgt und die Lösung auf das Tiergewebe aufgebracht wird.

9. Polyalkylencarbonat zur Verwendung in der Bildung eines in situ-Filmverbands nach Anspruch 3, wobei der Gehalt des Polyalkylencarbonats in der Suspension 10 Gew.-% bis 60 Gew.-%, bezogen auf die Suspension, beträgt und die Suspension auf das Tiergewebe aufgebracht wird.

10. Polyalkylencarbonat zur Verwendung nach Anspruch 1, wobei das fluide Polyalkylencarbonat auch ein Medikament enthält.

11. Polyalkylencarbonat zur Verwendung nach Anspruch 10, wobei das Medikament aus der Gruppe ausgewählt ist, welche aus antibakteriellen Mitteln, Steroiden, antifungalen Wirkstoffen, Immunomodulatoren, antimikrobiellen Mitteln, Steroiden, Mitteln gegen Akne und Mitteln gegen Psoriasis besteht.

12. Polyalkylencarbonat zur Verwendung in der Bildung eines Filmverbands auf Tiergewebe nach Anspruch 1, welches ein Polyalkylencarbonat mit einer Glasübergangstemperatur (Tg) von 15 bis 40°C in einer Fluidform umfasst, wobei das fluide Polyalkylencarbonat an Tiergewebe anhaftet.

13. Polyalkylencarbonat zur Verwendung nach Anspruch 12, wobei das Polyalkylencarbonat in Fluidform platziert wird, indem das Polyalkylencarbonat auf eine Temperatur von mindestens so hoch wie seine Glasübergangstemperatur erhöht wird.

14. Polyalkylencarbonat zur Verwendung nach Anspruch 5, wobei die Alkylenkomponente des Polyalkylencarbonats Ethylen ist und das biokompatible Lösungsmittel für Polyethylencarbonat Methylenchlorid umfasst.

15. Polyalkylencarbonat zur Verwendung nach Anspruch 5, wobei die Alkylenkomponente des Polyalkylencarbonats Propylen ist und das biokompatible Lösungsmittel für Polypropylencarbonat Aceton umfasst.

16. Polyalkylencarbonat zur Verwendung nach Anspruch 5, wobei das Polyalkylencarbonat Polyethylencarbonat mit einer Glasübergangstemperatur von 20 bis 25°C ist, das biokompatible Lösungsmittel Methylenchlorid ist und das Polyethylencarbonat in der Lösung in einer Konzentration von 10 bis 15 Gew.-%, bezogen auf die Lösung, vorhanden ist.

## Revendications

1. Polycarbonate d'alkylène pour l'utilisation dans la formation d'un pansement sous forme de film *in situ* pour le soin d'une blessure sur un tissu animal, qui comprend la préparation d'un fluide d'application comprenant un polycarbonate d'alkylène, l'application du fluide sur un tissu animal et la formation ainsi d'un film sur le tissu animal.

2. Polycarbonate d'alkylène pour l'utilisation dans la formation d'un pansement sous forme de film *in situ* selon la revendication 1, dans lequel le polycarbonate d'alkylène est mis sous forme de fluide par élévation du polycarbonate d'alkylène à une température au moins aussi élevée que sa température de transition vitreuse et pression du polycarbonate d'alkylène fluide sur le tissu animal.

3. Polycarbonate d'alkylène pour l'utilisation dans la formation d'un pansement sous forme de film *in situ* selon la revendication 1, dans lequel le polycarbonate d'alkylène est mis sous forme de fluide par formation d'une suspension du polycarbonate d'alkylène dans un milieu biocompatible, application du polycarbonate d'alkylène fluide sur le tissu animal et l'évaporation du milieu biocompatible.

4. Polycarbonate d'alkylène pour l'utilisation dans la formation d'un pansement sous forme de film *in situ* selon la revendication 1, dans lequel le polycarbonate d'alkylène est mis sous forme de fluide par formation une émulsion du polycarbonate d'alkylène dans un milieu biocompatible, application du polycarbonate d'alkylène fluide sur le tissu animal et l'évaporation du milieu biocompatible.

5. Polycarbonate d'alkylène pour l'utilisation dans la formation d'un pansement sous forme de film in situ selon la revendication 1, dans lequel le polycarbonate d'alkylène est mis sous forme de fluide par formation d'une solution de polycarbonate d'alkylène dans un solvant biocompatible, application du polycarbonate d'alkylène fluide sur le tissu animal et l'évaporation du solvant biocompatible.

6. Polycarbonate d'alkylène pour l'utilisation dans la formation d'un pansement sous forme de film *in situ* selon la revendication 1, dans lequel le constituant alkylène du polycarbonate d'alkylène contient de deux à neuf atomes de carbone.

7. Polycarbonate d'alkylène pour l'utilisation dans la formation d'un pansement sous forme de film *in situ* selon la revendication 1, dans lequel le constituant alkylène du polycarbonate d'alkylène est choisi dans le groupe constitué par l'éthylène, le propylène et le butylène.

8. Polycarbonate d'alkylène pour l'utilisation dans la formation d'un pansement sous forme de film *in situ* selon la revendication 5, dans lequel la teneur en polycarbonate d'alkylène dans la solution est de 5 % à 50 % en poids par rapport à la solution, et la solution est appliquée sur le tissu animal.

9. Polycarbonate d'alkylène pour l'utilisation dans la formation d'un pansement sous forme de film *in situ* selon la revendication 3, dans lequel la teneur en polycarbonate d'alkylène dans la suspension est de 10 % à 60 % en poids par rapport à la suspension, et la suspension est appliquée sur le tissu animal.

10. Polycarbonate d'alkylène pour l'utilisation selon la revendication 1, dans lequel le polycarbonate d'alkylène fluide contient aussi un médicament.

11. Polycarbonate d'alkylène pour l'utilisation selon la revendication 10, dans lequel le médicament est choisi dans les groupes constitués par les agents antibactériens, les stéroïdes, les agents antifongiques, les immunomodulateurs, les agents antimicrobiens, les stéroïdes, les agents antiacnéiques et les agents antipsoriasis.

12. Polycarbonate d'alkylène pour l'utilisation dans la formation d'un pansement sous forme de film sur un tissu animal selon la revendication 1, qui comprend un polycarbonate d'alkylène ayant une température de transition vitreuse (Tg) de 15 à 40 °C sous une forme fluide, lequel polycarbonate d'alkylène fluide adhère au tissu animal.

13. Polycarbonate d'alkylène pour l'utilisation selon la revendication 12, dans lequel le polycarbonate d'alkylène est mis sous forme de fluide par élévation du polycarbonate d'alkylène à une température au moins aussi élevée que sa température de transition vitreuse.

14. Polycarbonate d'alkylène pour l'utilisation selon la revendication 5, dans lequel le constituant alkylène du polycarbonate d'alkylène est l'éthylène et le solvant biocompatible pour le polycarbonate d'éthylène comprend du chlorure de méthylène.

15. Polycarbonate d'alkylène pour l'utilisation selon la revendication 5, dans lequel le constituant alkylène du polycarbonate d'alkylène est le propylène et le solvant biocompatible pour le polycarbonate de propylène comprend de l'acétone.

16. Polycarbonate d'alkylène pour l'utilisation selon la revendication 5, dans lequel le polycarbonate d'alkylène est un polycarbonate d'éthylène ayant une température de transition vitreuse de 20 à 25 °C, le solvant biocompatible est le chlorure de méthylène et le polycarbonate d'éthylène est présent dans la solution en une concentration de 10 à 15 % en poids par rapport à la solution.
